(19) Europäisches Patentamt
European Patent Office
Office européen des brevets

(11) Publication number: **0 207 701 A2**

(12) **EUROPEAN PATENT APPLICATION**

(21) Application number: 86304778.3

(22) Date of filing: 20.06.86

(51) Int. Cl.[4]: C 07 D 301/19
C 07 D 303/32, C 07 D 303/48

(30) Priority: 02.07.85 GB 8516744

(43) Date of publication of application: 07.01.87 Bulletin 87/2

(84) Designated Contracting States: BE DE FR GB IT NL SE

(71) Applicant: The British Petroleum Company p.l.c.
Britannic House Moor Lane
London EC2Y 9BU(GB)

(72) Inventor: Morris, George Ernest
The British Petroleum Company p.l.c. Chertsey Road
Sunbury-on-Thames Middlesex, TW16 7LN(GB)

(74) Representative: Fawcett, Richard Fennelly et al,
BP INTERNATIONAL LIMITED Patents Division Chertsey Road
Sunbury-on-Thames Middlesex, TW16 7LN(GB)

(54) **Process for the preparation of epoxides.**

(57) A process for preparing alpha substituted epoxides is provided. The process comprises contacting alcohols of formula $(R^2)(R^1)(HO)C-CG(R^3)X$ with a dihydrocarbyl peroxide, a copper compound and a Lewis base to yield compounds of formula

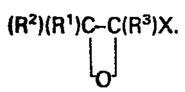

$R^1$, $R^2$, $R^3$ and $R^4$ can be hydrogen, alkyl, cycloalkyl or aromatic radicals whilst X is selected from $COR^4$, CN $COOR^4$ or $CONR^4_2$ groups. The process can be used for converting diacetone alcohol to its corresponding alpha substituted epoxide.

EP 0 207 701 A2

# PROCESS FOR THE PREPARATION OF EPOXIDES

The present invention relates to the catalysed preparation of alpha-substituted epoxides from alcohols. More specifically, this invention relates to the preparation of alpha-substituted epoxides by the catalysed oxidation of substituted alcohols using a dihydrocarbyl peroxide as the oxidising agent.

Alpha substituted epoxides find use as prescursors to certain food additives and as coal flotation agents.

GB patent 1381012 and A New Catalytic Route to Alpha-Ketoepoxides, Annali de Chemica 65, 1975 teach a process for the preparation of alpha-substituted epoxide compounds prepared by the copper catalysed oxidation of beta-alcohols using oxygen as the oxidising agent. However, a problem associated with the use of molecular oxygen is the formation of water which decreases the yield of product.

It has now been found that the disadvantage associated with the use of molecular oxygen and the production of water can be minimised by employing a dihydrocarbyl peroxide as the oxidising agent. In addition, this process avoids the use of molecular oxygen with its associated disadvantages such as the high risk of explosion. Moreover, the use of the peroxide oxidant makes the catalyst more efficient decreasing the amount of copper employed and also increases the overall rate of reaction.

Accordingly, the present invention provides a process for the production of alpha-substituted epoxides of formula (I) by contacting alcohols of formula (II) with a dihydrocarbyl peroxide in

the presence of a copper compound and a Lewis base at a temperature in the range 20 to 200°C.

The alpha-substituted epoxides prepared in accordance with the present invention have the following general formula:

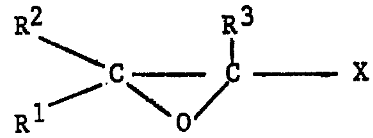 (I)

wherein each of $R^1$, $R^2$ and $R^3$ may each independently be a hydrogen atom, an unsubstituted or substituted alkyl, cycloalkyl or aromatic radical containing up to 12 carbon atoms and X is a group selected from $COR^4$, CN, $COOR^4$, or $CONR^4{}_2$ wherein $R^4$ is an atom or group as defined for $R^1$ $R^2$ and $R^3$. Preferably, $R^1$, $R^2$ and $R^3$ are either hydrogen or a $C_1$ to $C_4$ unsubstituted alkyl and X is a group selected from $COR^4$ wherein $R^4$ is as defined above. Examples of alpha-substituted epoxides which may be prepared by the process of the present invention include but are not limited to 3,4 epoxy-4- methyl-2-pentanone; 3,4 epoxy-2-pentanone; and cyclohexylidenecyclo- hexanone oxide.

The substituted alcohols used as the reactant in the present process have the following general formula

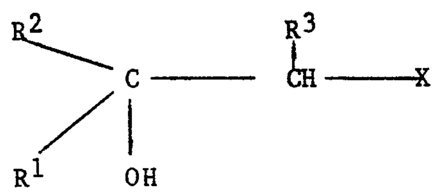 (II)

wherein $R^1$, $R^2$, $R^3$ and X are as defined above. It is preferred that both $R^1$ and $R^2$ are not hydrogen atoms. Preferably the substituted alcohol is a beta-substituted keto alcohol for example diacetone alcohol.

The inventive process is preferably conducted in the liquid phase in the presence of a copper compound, a Lewis base and a dihydrocarbyl peroxide. The copper compound is typically a copper compounds such as a copper halide, nitrate, acetate, benzoate, perchlorate and the like. Both copper (I) and copper (II) compounds can be used. The Lewis base may be substituted or unsubstituted and should be capable of complexing with copper.

Examples of suitable Lewis bases include pyridine, piperidine, phosphines, arsines, stibines, imidazoles and the like. Other coordinating ligands can also be used in place of the Lewis base which can complex with copper. Contemplated equivalent ligands which will perform substantialy the same as the Lewis base include nitriles and halide (e.g. chloride and bromide) ions. Preferred bases are the nitrogen-containing bases such as pyridine and alkyl substituted pyridines and imidazoles.

Unlike the prior art, dihydrocarbyl peroxide is used as the oxidising agent in the present process. The term "hydrocarbyl" is defined herein as the moiety formed by the removal of a hydrogen atom from a hydrocarbon. Accordingly, the hydrocarbyl radical of the dihydrocarbyl peroxide may suitably be an alkyl, aryl, alkaryl or aralkyl group having up to 12 carbon atoms. The hydrocarbyl radicals in the dihydrocaryl peroxide may be the same or different. Illustrative examples of suitable peroxides include but are not limited to di-cumyl peroxides and di-tertiary-butyl peroxides (DTBP), with DTBP being preferred. Since the dihydrocarbyl peroxide is used as the oxidising agent herein, it is preferred to conduct the present process in the substantial absence of molecular oxygen.

As mentioned above, the process is preferably conducted in a liquid phase and suitable solvents may be employed. Suitable solvents are polar solvents with $C_1$ to $C_4$ alcohol being preferred. The most preferred solvent is methanol. It is also possible to operate the present process in the absence of solvents in which case the reactant alcohol itself will act as the reaction medium.

The process conditions used in the present process can vary widely. The temperature can range from 20°C to 200°C with 40°C to 100°C being preferred. The pressure can also vary widely and is suitably from 1 to 30 atmospheres.

The concentration of copper compound, Lewis base and dihydrocarbyl peroxide can vary. Generally, the mole ratios of copper:dihydrocarbonyl peroxide:alcohol:Lewis base: will be in the range of 1:1-1000: 1-1000: 1-100 with the preferred ratios in the range of 1:1-100: 1-100: 1-10.

The present invention is illustrated by the following examples. However, these examples should not be construed as limiting the scope of this invention which includes equivalent modifications, variations and embodiments.

Example 1

A round 2-neck Pyrex flask fitted with a thermometer, reflux condenser and stirrer bar was charged with methanol (16.4g) di-tertiary butyl peroxide (14.6g) diacetone alcohol (13.8g) pyridine (1.3g) and meta-xylene (5.1g, an internal standard for GC analysis). The system was flushed with nitrogen then heated to reflux (70°C) in an oil bath, with stirring, over a period of 20min. The solution was maintained at reflux for 20min and GC analysis showed that no reaction had occurred during this time. Copper (I) chloride (0.21g) was then added, dissolving over about 10min to give a green solution which darkened progressively to brown. GC analysis of a sample taken 45min after the copper (I) chloride had been added showed the presence of unreacted DTBP (5.6g), diacetone alcohol (6.4g) acetone (0.7g) and the alpha-substituted epoxide (6.7g). This represents a selectively to the alpha-substituted epoxide based on DTBP consumed of 95% and a turnover rate of 39 moles alpha-keto epoxide per mole of copper (I) chloride per hour.

Example 2

A round 2-neck Pyrex flask fitted with a thermometer, reflux condenser and stirrer bar was charged with ditertiary butyl peroxide (13.0g) diacetone alcohol (25.1g) pyridine (1.3g) and meta-xylene (3.0g, an internal standard for GC analysis). The system was flushed with nitrogen then heated to 77°C in an oil bath, with stirring. Copper (I) chloride (0.25g) was then added, dissolving to give a green solution which darkened progressively to brown. The reaction mixture was maintained between 78°C and 82°C. GC analysis of a sample taken after 3 hours showed the presence of unreacted DTBP (1.8g), diacetone alcohol, acetone and the alpha-substituted epoxide (5.8g).

Example 3

A reaction vessel was charged with methanol (19.0 g) di-tertiary butyl peroxide (8.0 g) diacetone alcohol (10.0 g) 4-methyl pyridine (2.9 g), tetrahydropyran (2.1 g, internal standard for gc) and copper (I) chloride (0.30 g). The solution was stirred under nitrogen and heated to 48°C. An analysis of a sample after 4 hours showed formation of the alpha substituted epoxide (3.4 g) and the presence of unreacted ditertiary butyl peroxide (3.4 g).

Example 4

Example 3 was repeated but with 2 methyl pyridine (2.9 g) in place of 4-methyl pyridine. An analysis after 5½ hours showed alpha substituted epoxide (2.1 g) and di-tertiary butyl peroxide (4.6 g).

Example 5

Example 3 was repeated but with N-methyl imidazole (2.6 g) in place of 4-methyl pyridine. An analysis after 4½ hours showed alpha substituted epoxide (2.1 g) and di-tertiary butyl peroxide (4.3 g).

Example 6

Example 3 was repeated but with copper (I) bromide (0.44 g) in place of copper (I) chloride. An analysis after 4½ hours showed alpha substituted epoxide (2.0 g) and di-tertiary butyl peroxide (4.2 g).

Example 7

A reaction vessel was charged with methanol (19.0 g) di-cumyl peroxide (13.4 g) diacetone alcohol (6.8 g) pyridine (0.7 g) and copper (I) chloride (0.11 g). The mixture was heated with stirring to 60°C. An analysis of a sample taken after 35 mins showed the alpha substituted epoxide (2.1 g) and di-cumyl peroxide (4.9 g).

Claims:

1. A process for the production of alpha-substituted expoxides of formula

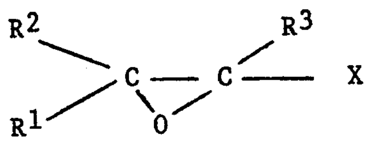

by contacting alcohols of formula,

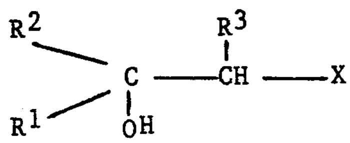

wherein in both formulae $R^1$, $R^2$ and $R^3$ are independently hydrogen, an unsubstituted or substituted alkyl, cycloalkyl or aromatic radical containing up to 12 carbon atoms and X is a group selected from $COR^4$, CN, $COOR^4$ and $CONR^4{}_2$ wherein $R^4$ is an atom or groups as defined for $R^1$, $R^2$ and $R^3$, with a dihydrocarbyl peroxide in the presence of a copper compound and a Lewis base at a temperature in the range 20 to 200°C.

2. A process as claimed in claim 1 wherein the alcohol is a beta-substituted keto alcohol.

3. A process as claimed in claim 2 wherein the alcohol is diacetone alcohol.

4. A process as claimed in claim 3 wherein the process is carried out in the presence of methanol.

5. A process as claimed in claim 4 wherein the Lewis base is

selected from pyridine, alkyl substituted pyridines and imidazoles.

6. A process as claimed in claim 5 wherein the mole ratios of copper compound:dihydrocarbyl peroxide:alcohol: Lewis base are in the range 1:1-100:1-100:1-10.

7. A process as claimed in claim 6 which is carried out in the substantial absence of oxygen.

8. A process as claimed in claim 7 wherein the dihydrocarbyl peroxide is either di-tertiary butyl peroxide or di-cumyl peroxide.